# FASCICULE DE BREVET EUROPEEN

(11) **EP 1 011 312 B1**
(45) Date de publication et mention de la délivrance du brevet: **31.03.2004**
(21) Numéro de dépôt: 98904224.7
(22) Date de dépôt: 27.01.1998
(51) Int. Cl.: A01H 15/00, A01G 1/04

(54) **OBTENTION DE NOUVEAUX HYBRIDES D'AGARICUS BISPORUS**
VERFAHREN FÜR DIE ERHALTUNG VON NEUEN HYBRIDEN VON AGARICUS BISPORUS
METHOD FOR PRODUCING NOVEL AGARICUS BISPORUS HYBRIDS

(30) Priorité: 27.01.1997 FR 9700834
(43) Date de publication de la demande: 28.06.2000
(73) Titulaire: INSTITUT NATIONAL DE LA RECHERCHE AGRONOMIQUE (INRA), 75338 Paris Cédex 07 (FR); C.T.C.- CENTRE TECHNIQUE DU CHAMPIGNON, 49400 Distre (FR)
(72) Inventeur: MOQUET, Frédéric, F-33125 Saint Magne (FR); OLIVIER, Jean-Marc, F-33640 Beautiran (FR); VEDIE, Régis, F-37370 Saint Paterne Racan (FR); CALLAC, Philippe, F-33140 Villenave d'Ornon (FR)
(74) Mandataire: Texier, Christian
(86) Numéro de dépôt international: PCT/FR1998/000139
(87) Numéro de publication internationale: WO 1998/032327

(56) Documents cités:
- FR-A- 2 696 187
- US-A- 4 996 390
- US-A- 5 304 721
- PAHIL, V ET AL.: "The testing and improvement of high temperature, wild Agaricus strains for use in tropical and sub-tropical climates" PROCEEDINGS OF THE 13TH INTERNATIONAL CONGRESS OF THE SCIENCE AND CULTIVATION OF EDIBLE FUNGUI , vol. 2, 1 - 6 septembre 1991, pages 589-599, XP002046576 DUBLIN
- HORNA, S. ET AL.: "Selection of lines of Agaricus brinnescens Peck for higher production temperatures" HORTSCIENCE, vol. 18, no. 6, 1983, pages 866-868, XP002046577
- FLEGG, P.: "Effect of temperature on mushroom Agaricus bisporus: a brief review of twenty years of research" MUSHROOM JOURNAL, vol. 1980, no. 86, 1980, pages 65-67, XP002046578

## Description

L'invention a pour objet un procédé d'obtention de nouveaux hybrides d'*Agaricus bisporus*.

On sait que la culture du champignon *Agaricus bisporus*, également appelé champignon de couche, comporte un certain nombre d'étapes qui sont rappelées ci-après.

Une première étape (compostage) consiste en la préparation d'un substrat à base de paille, de fumier de cheval, et éventuellement d'additifs azotés, en vue d'obtenir par fermentation un substrat (compost) contenant des éléments nutritifs utilisables par le mycélium en phase de multiplication. La durée de cette étape de compostage est de l'ordre de 2 à 3 semaines. Généralement, on complète l'étape de compostage par une étape dite de pasteurisation qui consiste à améliorer la qualité du compost par fermentation dirigée dans des salles spéciales, généralement pendant 5 à 7 jours. L'étape suivante est le lardage qui consiste à ensemencer le substrat de culture ainsi obtenu avec du "blanc", c'est-à-dire du mycélium multiplié sur grains de céréales. Le taux de lardage désigne le rapport du poids de blanc au poids de compost. Ce taux de lardage est généralement supérieur à 0,5 % et inférieur à 1 %. Vient ensuite l'étape d'incubation pendant laquelle le mycélium se multiplie et colonise progressivement le substrat de culture. Cette phase d'incubation est réalisée soit dans un local spécial climatisé, soit dans une galerie de cave de culture, avec une température de substrat de l'ordre de 24-26°. La durée moyenne de la phase d'incubation est de 14 jours environ. On effectue ensuite l'opération dite de gobetage, qui consiste à recouvrir le substrat de culture avec une couche d'un matériau de gobetage généralement constitué, en France notamment, par un mélange de calcaire (par exemple du tuffeau) et de tourbe. Cette couche a une épaisseur qui est généralement de l'ordre de 3 à 5 cm. Pour favoriser la fructification, on utilise une technique de choc thermique qui consiste à abaisser la température ambiante vers 16-17°C avec un renouvellement de l'air important et des arrosages réguliers. Cette dernière phase d'induction de la fructification est parfois précédée, juste après le gobetage, d'une phase dite de pré-fructification, qui consiste à maintenir pendant 8 à 10 jours une température ambiante voisine de 24°C avec faible renouvellement de l'air.

La récolte des champignons se fait à partir de levées successives (appelées "volées"). La récolte dite de première volée peut être effectuée généralement trois semaines après le gobetage. On peut généralement récolter de 4 à 5 volées sur une période de 5 à 7 semaines après le début de la première récolte. Le rendement total de la récolte peut être exprimé en poids de champignons pour 100 kg de compost. Les rendements moyens se situent aux environs de 30 %.

Ainsi, la culture du champignon de couche est très contraignante, notamment en ce qui concerne la régulation de la température qui entraîne une consommation d'énergie notable, et donc des frais importants.

L'opération de gobetage est également coûteuse en main-d'oeuvre et en matériau de gobetage.

L'opération de gobetage est cependant indispensable pour obtenir une fructification du champignon de couche. Il en est de même pour l'opération de choc thermique.

Sur les méthodes de culture du champignon de couche, on peut citer notamment les ouvrages suivants : J. Delmas, Les champignons et leur culture, publié par La Maison Rustique - Flammarion (Paris, France) ; The cultivation of Mushrooms (1988) publié par les éditions Somycel ; et Olivier J. M. et al., La culture des champignons (1991) publié par les éditions Armand Colin. Sur l'opération de gobetage et sa nécessité pour obtenir des fructifications, on peut citer en outre W. A. Hayes, The Casing Layer, publié par W. S. Maney and Son Ltd., Leeds (Grande-Bretagne),

Des méthodes de culture sont également décrites dans les documents US 5 304 721, XP 002 046 577 (S.L. Horna, D.J. Royse-Hort Science, Vol 18(6), 1983).

Les matériaux utilisés pour le gobetage sont très divers. On peut citer, outre la tourbe et les mélanges de tourbe et de roches calcaires, l'argile, le gypse, le sable, etc. On a également préconisé l'utilisation de nappes de matériaux synthétiques, ou des matériaux polymères naturels ou synthétiques capables de retenir de l'eau. On peut citer à ce sujet notamment les brevets ou demandes de brevet FR-2 549 346, FR-2 603 767, FR-2 676 158, FR-2 723 506 et US-4 079 543.

On a maintenant découvert qu'il est possible d'obtenir des Agaricus hybrides qui peuvent fructifier sans gobetage et qui peuvent être cultivés dans des conditions peu contraignantes en ce qui concerne la régulation de la température. On a découvert en effet que certains hybrides obtenus par croisement de souches d'*Agaricus bisporus* avec des *Agaricus bisporus* de la variété *burnettii,* ou des descendants ou des produits de croisement de cette variété, sont capables de fructifier sans gobetage et peuvent en outre fructifier sans choc thermique. En outre l'invention permet d'obtenir des hybrides dont les mycéliums sont capables de se multiplier sur des substrats ayant des températures élevées, sans perdre leur aptitude à la fructification.

La culture de ces nouveaux hybrides peut donc être mise en oeuvre en réduisant de façon importante les coûts en main d'oeuvre et en énergie.

Grâce à l'invention, il devient en outre possible de cultiver les champignons de couche même dans les pays à climat chaud où cette culture ne pouvait pas jusqu'à présent être effectuée de façon rentable.

L'invention a pour objet un procédé selon la revendication 1.

L'expression "mycélium hybride" désigne, dans la présente demande :
a) soit un mycélium issu d'au moins un croisement entre deux souches parentales d'*Agaricus bisporus*, c'est-à-dire issu de l'association par fusion des cytoplasmes (plasmogamie) de deux homocaryons eux-mêmes issus de deux souches hétérocaryotiques parentales distinctes,
b) soit un mycélium issu d'une spore produite par les fructifications d'un mycélium tel que défini en a) ci-dessus,
c) soit un mycélium issu d'un protoplaste obtenu à partir d'un mycélium tel que défini en a) et/ou en b) ci-dessus.

Les mycéliums de l'invention sont donc des hybrides intraspécifiques.

Lorsque le mycélium hybride de l'invention est capable de croître sur un substrat de culture usuel à une température supérieure ou égale, par exemple, à 30°C, il faut comprendre qu'il est capable d'envahir ledit substrat à cette température, et cela, bien entendu, sans perte d'aptitude à la fructification, ce qui signifie notamment, que la fructification se produit dans des délais normaux (pas plus de 5 à 6 semaines après le lardage), que le rendement pondéral (poids de champignons récoltés pour 100 kg de compost), sur 5 semaines de récolte, est au moins égal à 10 %, et que les champignons produits ont une conformation (morphologie, dimensions) normale.

Les substrats de culture usuels sont bien connus, et sont décrits par exemple dans les ouvrages mentionnés ci-dessus. Il s'agit généralement de composts à base de fumier de cheval et de paille.

Le procédé de l'invention tel que défini dans la renvendication 1 concerne notamment un mycélium hybride capable de fructifier à une température de substrat supérieure ou égale à 28°C et/ou capable de fructifier à une température ambiante supérieure ou égale à 26°C et en particulier supérieure ou égale à 28°C, et/ou capable de se multiplier sur un substrat de culture usuel et d'envahir ledit substrat à une température de substrat supérieure ou égale à 32°C, sans perte d'aptitude à la fructification, et/ou capable de fructifier sans gobetage avec un rendement en sporophores d'au moins 10 kg pour 100 kg de substrat de culture sur une période de 5 semaines à compter du début de la fructification.

Le mycélium hybride de l'invention est généralement hétérocaryotique, mais il peut aussi être issu d'une culture monospore hétérocaryotique ou même homocaryotique d'un tel hybride. On a en effet découvert que, de façon surprenante, certains mycéliums homocaryotiques (c'est-à-dire haploïdes) issus d'hybrides selon l'invention étaient capables de fructifier ; voir la partie expérimentale ci-après.

L'invention s'étend aux sporophores issus de la fructification d'un mycélium hybride tel que défini précédemment, ainsi qu'à des hétérocaryons ou homocaryons issus d'un tel mycélium ou d'un tel sporophore.

Les modes d'exécution particuliers sont décrits dans les revendications dépendantes.

L'invention concerne également un procédé d'obtention d'un mycélium hybride, de sporophores ou d'hétérocaryons ou d'homocaryons tels que définis précédemment, ce procédé étant caractérisé par le fait qu'il comprend au moins une étape de croisement entre une souche d'*Agaricus bisporus* ou un descendant hybride de ladite souche et un *Agaricus bisporus* de la variété *burnettii* ou un descendant hybride de ladite variété, et par le fait que l'on sélectionne, parmi les produits de croisement, ceux qui présentent le caractère phénotypique recherché. Il résulte de cela que ces caractères phénotypiques sont transmissibles génétiquement, et sont exprimés de façon constante après qu'ils ont été transmis.

Le caractère phénotypique recherché peut être soit l'un des caractères phénotypiques mentionnés dans la définition du mycélium hybride de l'invention, soit une combinaison de deux ou plus de ces caractères.

La variété *burnettii* de l'espèce *Agaricus bisporus* a été décrit par Callac et al., Mycologia 85(5), 835-851 (1993).

Les étapes de croisement peuvent être réalisées de façon connue en soi. Elles sont facilitées par le fait que les *Agaricus bisporus* var. *burnettii* lorsqu'ils sont croisés avec une souche quelconque d'*Agaricus bisporus*, fournissent des hybrides dont les spores sont majoritairement homocaryotiques ; voir à ce propos le document WO 94/07357 et le brevet US 5304721.

Pour la mise en oeuvre d'une étape de croisement, dans le procédé d'obtention qui vient d'être décrit, on peut utiliser comme *Agaricus bisporus* de la variété *burnettii* notamment la souche JB3 ms, déposée à la CNCM (France) le 23 janvier 1997 sous le numéro 1-1811, et la souche JB162 ms déposée à la CNCM le 23 janvier 1997 sous le numéro I-1812.

Comme premier *Agaricus bisporus* (tel que défini dans le procédé d'obtention décrit ci-dessus) on peut utiliser par exemple un *Agaricus bisporus* n'appartenant pas à la variété *burnettii*, ou un descendant hybride d'un tel *Agaricus bisporus.* On peut utiliser notamment différentes souches de champignon qui sont disponibles commercialement, comme par exemple HU1. La souche HU1, également appelée U1, est une souche commerciale obtenue par la Station de Horst (Pays-Bas) et diffusée par Sylvan-Somycel. Ledit premier *Agaricus bisporus* peut aussi appartenir à la variété *burnettii*, ou être un descendant hybride de ladite variété.

Pour la mise en oeuvre des plans de croisement, on peut utiliser notamment les méthodes décrites dans le document PCT WO 94/07357, correspondant notamment à la demande de brevet USA N° 08/403750.

Les exemples suivants illustrent l'invention.

### EXEMPLES

### Exemple 1 :

On a isolé selon la méthode des protoplastes, un premier homocaryon *d'Agaricus bisporus* de la variété HORST® U1 commercialisée par la Société Sylvan-Somycel.

Cet homocaryon a été multiplié de manière usuelle sur compost usuel pour obtenir un mycélium homocaryotique, puis a été croisé avec un mycélium homocaryotique issu de la souche JB3. L'hétérocaryon obtenu a été mis en culture sur compost usuel. Après 14 jours d'incubation à 24°C, on procède à un gobetage avec un mélange de tuffeau pulvérisé et de tourbe (2:1 en volume). Le tuffeau est un calcaire naturel.

La culture est placée en salle de fructification à 16°C. Les sporophores apparaissent 16 jours après le gobetage.

L'hybride de première génération obtenu fournit des fructifications dont les basides sont majoritairement tétrasporées. Les spores récupérées sont mises à germer sur milieu Raper (référence : Raper et al. (1972) Mycologia, 64, 1088-1117). Les mycéliums issus des spores germées ont été isolés et leur caractère homocaryotique a été vérifié par des tests de confrontation avec des homocaryons testeurs et par des analyses génotypiques à l'aide de marqueurs enzymatiques et moléculaires.

Un descendant monospore homocaryotique de cet hybride tétrasporé a été croisé avec un second homocaryon de la souche U1, ledit second homocaryon ayant une polarité opposée à celle du premier homocaryon mentionné ci-dessus et utilisé précédemment comme parent de la souche hybride de première génération.

On obtient ainsi un hybride de seconde génération, dont les fructifications, obtenues comme précédemment, sont tétrasporées.

On a isolé et mis en germination, comme précédemment, 112 spores issues de la fructification de l'hybride de seconde génération.

Les mycéliums obtenus ont été utilisés pour faire des croisements avec un mycélium homocaryotique compatible issu d'une monospore de la souche JB162 ms.

On a ainsi obtenu une troisième génération d'hybrides.

### Etude des hybrides de 3ème génération

Des premiers essais ont porté sur 36 hybrides de 3ème génération.

Les essais ont été menés dans des caisses contenant 15 kg de compost usuel (fumier de cheval + paille).

L'opération de lardage a été effectuée à un taux de 1 %.

La température du compost au moment du lardage était de 28°C.

La température ambiante pendant la période de croissance du mycélium et d'envahissement du compost était réglée à 25°C (humidité relative 95 %).

Une opération de gobetage à l'aide d'un matériau de gobetage usuel (calcaire et tourbe dans les proportions 3 : 1 en volume) a été effectuée 14 jours après le lardage.

Après l'opération de gobetage, la température ambiante a été abaissée à 16°C (humidité relative : 92 %).

On a observé des fructifications avant même d'avoir effectué le gobetage, et donc à une température ambiante de 25°C, pour 11 des 36 hybrides étudiés.

De nouvelles mises en culture des souches ayant fructifié sans gobetage ont permis de vérifier la répétabilité du phénomène observé, y compris sur des composts différents (par exemple fumier de cheval sans paille ajoutée) du compost utilisé dans les expériences précédentes.

Les souches parentes (hybride de deuxième génération et JB162 ms), cultivées dans les mêmes conditions, ne fructifient pas sans gobetage.

Dans d'autres essais portant sur 32 hybrides de troisième génération (dont certains avaient également été testés dans l'essai décrit ci-dessus), on a observé que 25 hybrides ont fructifié sans gobetage.

Dans plusieurs cas, le rendement en champignons des cultures effectuées sans gobetage, cumulé sur une période allant jusqu'à cinq semaines après le lardage, est supérieur au rendement cumulé des cultures des mêmes hybrides ayant subi une opération de gobetage deux semaines après le lardage.

On a en outre observé des fructifications sans gobetage sur des substrats dont la température était supérieure à 28°C et à des températures ambiantes également supérieure à 28°C.

Des fructifications sans gobetage ont également été observées, dans les mêmes conditions que précédemment, avec divers hybrides de troisième génération obtenus par croisement d'homocaryons de l'hybride de deuxième génération avec des mycéliums homocaryotiques issus de monospores de la souche JB162 ms autres que le mycélium homocaryotique utilisé pour obtenir les hybrides de troisième génération décrits précédemment.

D'autres hybrides de troisième génération obtenus par croisement de l'hybride de deuxième génération avec une souche d'*Agaricus bisporus* var. *burnetii* autre que JB162, ont également donné lieu à des fructifications sans gobetage, dans les mêmes conditions que précédemment.

Par ailleurs, on a observé que des mycéliums homocaryotiques issus de cultures monospores d'hybrides descendants de JB162 ms sont capables de fructifier après incubation sur compost usuel, à une température de compost de 28-30°C, avec ou sans gobetage.

L'état homocaryotique de ces mycéliums et de ces fructifications a été vérifié sur des marqueurs chromosomiques par RFLP, après amplification par PCR.

En outre, on a testé la croissance du mycélium de certains hybrides de troisième génération à une température de substrat ayant atteint 35°C une semaine après lardage. Le mycélium s'est bien développé et on a pu ultérieurement obtenir des fructifications sans gobetage, à une température ambiante de 25°C, à partir d'un tel mycélium qui s'est développé temporairement à 35°C.

On a également étudié l'effet d'un choc thermique froid. Des bacs de culture, contenant 7 kg de compost, ont été placés dans une chambre froide à 4°C, sept jours après le lardage, pendant 14 heures.

Pour les bacs traités par le froid, on a observé un jour de retard à la fructification par rapport aux bacs restés à 25°C. Le choc froid au cours de l'incubation n'a donc pas d'incidence notable sur l'aptitude à la fructification sans gobetage.

Par ailleurs, pour certains hybrides de troisième génération, on a procédé à des essais de réduction du taux de lardage (0,5 % ou 0,25 % au lieu de 1 %). Les rendements n'ont pas été affectés, mais la première récolte a été retardée de 4 à 6 jours, par rapport à la récolte obtenue avec un taux de lardage de 1 %.

Des essais comparatifs ont montré que l'hybride de première génération n'a pas d'aptitude à fructifier sans gobetage et que de même, des hybrides obtenus par croisement de l'hybride de deuxième génération avec le premier homocaryon de U1 n'ont pas d'aptitude à fructifier sans gobetage.

A titre de remarque générale, il faut noter enfin que lorsqu'on ne travaille pas dans des salles à atmosphère contrôlée, par exemple lorsqu'on effectue la culture en caves, l'absence de gobetage peut conduire à un dessèchement superficiel du compost en raison de l'évaporation. Un tel dessèchement peut être préjudiciable à la fructification. Il est toutefois possible de remédier à ce problème soit par arrosages modérés répétés, soit en recouvrant le compost d'un film plastique jusqu'à ce que les fructifications commencent à apparaître. On peut également recouvrir le substrat dans lequel s'est développé le mycélium d'une couche d'épaisseur réduite (par exemple de 0,5 à 2 cm), de matériau de gobetage.

### Exemple 2 :

On choisit une souche hybride de troisième génération obtenue à l'exemple précédent et qui fructifie sans gobetage. On la cultive et la fait fructifier.

On a isolé des spores issues des fructifications ainsi obtenues.

La majorité de ces spores étaient homocaryotiques. Ces spores homocaryotiques, après germination, ont été confrontées avec le premier homocaryon de la souche U1 utilisé pour l'obtention de l'hybride de première génération (voir exemple 1). On a ainsi obtenu des mycéliums hétérocaryotiques hybrides de quatrième génération.

Les hybrides de quatrième génération obtenus, ainsi que les souches issues de spores hétérocaryotiques de l'hybride de troisième génération choisi ci-dessus ont été cultivés en paniers contenant 6,5 kg de compost.

Les conditions de culture étaient les suivantes :
- Jour J0 : lardage (1 %).

Après le lardage, les cultures ont été maintenues à une température ambiante de 25°C (humidité relative : 90-93 %).
- Jour J0+14 : la température ambiante a été abaissée à 16°C (humidité relative : 90-92 %).

Les résultats ont été les suivants.

31 souches hybrides de quatrième génération, sur 82 testées, ont fructifié sans gobetage.

Dans d'autres expériences, les souches hybrides ont été cultivées comme précédemment, mais à température constante de 25°C (c'est-à-dire en omettant le refroidissement à 16°C au jour J0+14). On a encore observé des fructifications sans gobetage.

Ainsi, le caractère de fructification sans gobetage et à température élevée est transmissible génétiquement.

Par ailleurs, parmi les cultures monospores isolées de l'hybride de troisième génération étudié, on a trouvé 14 souches dont les mycéliums étaient de nature hétérocaryotique, et qui provenaient donc de spores hétérocaryotiques. Onze d'entre eux ont été étudiés. Sur ces 11 mycéliums, trois ont fructifié sans gobetage.

## Revendications

1. Procédé de culture d'*Agaricus bisporus,* comprenant une étape de multiplication de mycélium hybride sur un substrat de culture usuel avec envahissement dudit substrat, suivie d'une étape de fructification dans lequel le mycélium présente l'un au moins des caractères phénotypiques suivants :
- après multiplication sur un substrat de culture usuel et envahissement dudit substrat, il est capable de fructifier sans gobetage ;
- après multiplication sur un substrat de culture usuel et envahissement dudit substrat, il est capable de fructifier à une température de substrat supérieure ou égale à 26°C ;
- après multiplication sur un substrat de culture usuel et envahissement dudit substrat, il est capable de fructifier à un température ambiante supérieure ou égale à 25°C ;
- il est capable de croître sur un substrat de culture usuel à une température de substrat supérieure ou égale à 32°C,
**caractérisé en ce que** ledit procédé ne comporte pas d'étape de gobetage.

2. Procédé selon la revendication 1, **caractérisé en ce qu'**il présente l'une au moins des caractéristiques suivantes :
- on effectue la multiplication du mycélium sur le substrat avec envahissement dudit substrat à une température de substrat supérieure ou égale à 32°C,
- on effectue l'étape de fructification à une température de substrat supérieure ou égale à 26°C,
- on effectue l'étape de fructification à une température ambiante supérieure ou égale à 25°C.

3. Procédé selon la revendication 2, **caractérisé par le fait que** l'on effectue l'étape de fructification à une température de substrat supérieure ou égale à 28°C.

4. Procédé selon l'une quelconque des revendications 2 ou 3, **caractérisé par le fait que** l'on effectue l'étape de fructification à une température ambiante supérieure ou égale à 26°C.

5. Procédé selon la revendication 4, **caractérisé par le fait que** ladite température ambiante est supérieure ou égale à 28°C.

6. Procédé selon l'une quelconque des revendications 2 à 5, **caractérisé par le fait qu'**il ne comporte pas d'étape de choc thermique après l'étape de multiplication du mycélium.

7. Procédé selon l'une des revendications 1 à 6, **caractérisé en ce que** le mycélium est capable de fructifier à une température de substrat supérieure ou égale à 28°C.

8. Procédé selon l'une des revendications 1 à 7, **caractérisé en ce que** le mycélium est capable de fructifier à une température ambiante supérieure ou égale à 26°C.

9. Procédé selon la revendication 8, **caractérisé en ce que** le mycélium est capable de fructifier à une température ambiante supérieure ou égale à 28°C.

10. Procédé selon l'une des revendications 1 à 9, **caractérisé en ce que** le mycélium est capable de fructifier sans gobetage avec un rendement en sporophores d'au moins 10 kg pour 100 kg de substrat de culture, sur une période de 5 semaines à compter du début de la fructification.

11. Procédé selon l'une des revendications 1 à 10, **caractérisé en ce que** ledit mycélium est hétérocaryotique.

12. Procédé selon l'une des revendications 1 à 10, **caractérisé en ce que** ledit mycélium est homocaryotique.

13. Procédé selon l'une des revendications 1 à 12, **caractérisé en ce que** le mycélium est capable de fructifier.

14. Procédé d'obtention d'un mycélium tel que défini dans l'une quelconque des revendications 1 à 13, **caractérisé par le fait qu'**il comprend au moins une étape de croisement entre un premier *Agaricus bisporus* ou un descendant hybride de celui-ci et un *Agaricus bisporus* de la variété *burnettii*, ou un descendant hybride de ladite variété, et **par le fait que** l'on sélectionne, parmi les produits de croisement, ceux qui présentent le caractère phénotypique recherché.

15. Procédé selon la revendication 14, **caractérisé par le fait qu'**il comprend au moins une étape de croisement dans laquelle l'*Agaricus bisporus* de la variété *burnettii* est la souche déposée à la CNCM sous le n° I-1811.

16. Procédé selon l'une des revendications 14 et 15, **caractérisé par le fait qu'**il comprend au moins une étape de croisement dans laquelle l'*Agaricus bisporus* de la variété *burnettii* est la souche déposée à la CNCM sous le n° I-1812.

17. Procédé selon l'une quelconque des revendications 14 à 16, **caractérisé par le fait qu'**il comprend au moins une étape de croisement dans lequel ledit premier *Agaricus bisporus* est choisi parmi un *Agaricus bisporus* de la variété *burnettii*, un descendant hybride de ladite variété et une souche commerciale d'*Agaricus bisporus*.

18. Procédé selon la revendication 17, dans lequel ladite souche commerciale est la variété commerciale U1.

## Claims

1. Method of growing *Agaricus bisporus,* which comprises a step in which hybrid mycelium is multiplied on a usual culture substrate with invasion of the said substrate, followed by a step in which fruiting bodies are formed, in which the mycelium exhibits at least one of the following phenotypic traits:
- after multiplication on a usual culture substrate and invasion of the said substrate, it is capable of forming fruiting bodies without casing;
- after multiplication on a usual culture substrate and invasion of the said substrate, it is capable of forming fruiting bodies at a substrate temperature of 26°C or above;
- after multiplication on a usual culture substrate and invasion of the said substrate, it is capable of forming fruiting bodies at an ambient temperature of 25°C or above;
- it is capable of growing on a usual culture substrate at a substrate temperature of 32°C or above,
**characterized in that** the said method does not comprise a casing step.

2. Method according to Claim 1, **characterized in that** it exhibits at least one of the following traits:
- the step in which the mycelium is multiplied on the substrate with invasion of the said substrate is carried out at a substrate temperature of 32°C or above;
- the step in which fruiting bodies are formed is carried out at a substrate temperature of 26°C or above;
- the step in which fruiting bodies are formed is carried out at an ambient temperature of 25°C or above.

3. Method according to Claim 2, **characterized in that** the step in which fruiting bodies are formed is carried out at a substrate temperature of 28°C or above.

4. Method according to either of Claims 2 and 3, **characterized in that** the step in which the fruiting bodies are formed is carried out at an ambient temperature of 26°C or above.

5. Method according to Claim 4, **characterized in that** the said ambient temperature is 28°C or above.

6. Method according to any of Claims 2 to 5, **characterized in that** it does not comprise a temperature shock step after the step in which the mycelium is multiplied.

7. Method according to one of Claims 1 to 6, **characterized in that** the mycelium is capable of forming fruiting bodies at a substrate temperature of 28°C or above.

8. Method according to one of Claims 1 to 7, **characterized in that** the mycelium is capable of forming fruiting bodies at an ambient temperature of 26°C or above.

9. Method according to Claim 8, **characterized in that** the mycelium is capable of forming fruiting bodies at an ambient temperature of 28°C or above.

10. Method according to one of Claims 1 to 9, **characterized in that** the mycelium is capable of forming fruiting bodies without casing with a sporophore yield of at least 10 kg per 100 kg of culture substrate over a period of 5 weeks, starting at the beginning of the formation of fruiting bodies.

11. Method according to one of Claims 1 to 10, **characterized in that** the said mycelium is heterokaryotic.

12. Method according to one of Claims 1 to 10, **characterized in that** the said mycelium is homokaryotic.

13. Method according to one of Claims 1 to 12, **characterized in that** the mycelium is capable of forming fruiting bodies.

14. Method of obtaining a mycelium as defined in any one of Claims 1 to 13, **characterized in that** it comprises at least one hybridization step between a first *Agaricus bisporus* or a hybrid progeny of this strain and an *Agaricus bisporus* var. *burnettii* or a hybrid progeny of the said variety, and **in that** those hybridization products are selected which exhibit the desired phenotypic trait.

15. Method according to Claim 14, **characterized in that** it comprises at least one hybridization step in which Agaricus *bisporus* var. *burnettii* is the strain which has been deposited at CNCM under the No. 1-1811.

16. Method according to either of Claims 14 and 15, **characterized in that** it comprises at least one hybridization step in which *Agaricus bisporus* var. *burnettii* is the strain which has been deposited at CNCM under the No. 1-1812.

17. Method according to any one of Claims 14 to 16, **characterized in that** it comprises at least one hybridization step in which the said first *Agaricus bisporus* is chosen from an *Agaricus bisporus* var. *burnettii*, a hybrid progeny of the said variety and a commercial strain of *Agaricus bisporus*.

18. Method according to Claim 17, in which the said commercial strain is the commercial variety U1.

## Patentansprüche

1. Verfahren zur Kultivierung von *Agaricus bisporus*, umfassend einen Schritt einer Vermehrung von hybridem Myzel auf einem üblichen Kultursubstrat mit Durchwucherung des Substrats, gefolgt von einem Schritt einer Fruktifizierung, bei welchem das Myzel wenigstens eine der folgenden phänotypischen Eigenschaften aufweist:
- nach der Vermehrung auf einem üblichen Kultursubstrat und Durchwucherung des Substrats ist es in der Lage, ohne Überdeckung ("gobetage") zu fruktifizieren;
- nach der Vermehrung auf einem üblichen Kultursubstrat und Durchwucherung des Substrats ist es in der Lage, bei einer Substrattemperatur über oder gleich 26°C zu fruktifizieren;
- nach der Vermehrung auf einem üblichen Kultursubstrat und Durchwucherung des Substrats ist es in der Lage, bei einer Umgebungstemperatur über oder gleich 25°C zu fruktifizieren;
- es ist in der Lage, auf einem üblichen Kultursubstrat bei
einer Substrattemperatur über oder gleich 32°C zu wachsen, **dadurch gekennzeichnet, dass** das Verfahren keinen Schritt einer Überdeckung ("gobetage") umfasst.

2. Verfahren nach Anspruch 1, **dadurch gekennzeichnet, dass** es wenigstens eines der folgenden Merkmale aufweist:
- man führt die Vermehrung des Myzels auf dem Substrat mit Durchwucherung des Substrats bei einer Substrattemperatur über oder gleich 32°C aus,
- man führt den Fruktifizierungsschritt bei einer Substrattemperatur über oder gleich 26°C aus,
- man führt den Fruktifizierungsschritt bei einer Umgebungstemperatur über oder gleich 25°C aus.

3. Verfahren nach Anspruch 2, **dadurch gekennzeichnet, dass** man den Fruktifizierungsschritt bei einer Substrattemperatur über oder gleich 28°C ausführt.

4. Verfahren nach einem der Ansprüche 2 oder 3, **dadurch gekennzeichnet, dass** man den Fruktifizierungsschritt bei einer Umgebungstemperatur über oder gleich 26°C ausführt.

5. Verfahren nach Anspruch 4, **dadurch gekennzeichnet, dass** die Umgebungstemperatur über oder bei 28°C liegt.

6. Verfahren nach einem der Ansprüche 2 bis 5, **dadurch gekennzeichnet, dass** es keinen Schritt eines thermischen Schocks nach dem Schritt der Vermehrung des Myzels umfasst.

7. Verfahren nach einem der Ansprüche 1 bis 6, **dadurch gekennzeichnet, dass** das Myzel in der Lage ist, bei einer Substrattemperatur über oder gleich 28°C zu fruktifizieren.

8. Verfahren nach einem der Ansprüche 1 bis 7, **dadurch gekennzeichnet, dass** das Myzel in der Lage ist, bei einer Umgebungstemperatur über oder gleich 26°C zu fruktifizieren.

9. Verfahren nach Anspruch 8, **dadurch gekennzeichnet, dass** das Myzel in der Lage ist, bei einer Umgebungstemperatur über oder gleich 28°C zu fruktifizieren.

10. Verfahren nach einem der Ansprüche 1 bis 9, **dadurch gekennzeichnet, dass** das Myzel in der Lage ist, ohne Überdeckung ("gobetage") mit einer Ausbeute an Sporophoren von wenigstens 10 kg pro 100 kg Kultursubstrat über einen Zeitraum von 5 Wochen, berechnet ab dem Beginn der Fruktifizierung, zu fruktifizieren.

11. Verfahren nach einem der Ansprüche 1 bis 10, **dadurch gekennzeichnet, dass** das Myzel heterokaryotisch ist.

12. Verfahren nach einem der Ansprüche 1 bis 10, **dadurch gekennzeichnet, dass** das Myzel homokaryotisch ist.

13. Verfahren nach einem der Ansprüche 1 bis 12, **dadurch gekennzeichnet, dass** das Myzel in der Lage ist, zu fruktifizieren.

14. Verfahren zur Gewinnung eines Myzels, wie in einem der Ansprüche 1 bis 13 definiert, **dadurch gekennzeichnet, dass** es wenigstens einen Schritt einer Kreuzung zwischen einem ersten *Agaricus bisporus* oder einem hybriden Abkömmling von diesem und einem *Agaricus bisporus* der Varietät *burnettii* oder einem hybriden Abkömmling von dieser Varietät umfasst, und dadurch, dass man unter den Kreuzungsprodukten jene auswählt, die die gesuchte phänotypische Eigenschaft aufweisen.

15. Verfahren nach Anspruch 14, **dadurch gekennzeichnet, dass** es wenigstens einen Schritt einer Kreuzung umfasst, in welchem der *Agaricus bisporus* der Varietät *burnettii* der bei der CNCM unter der Nr. 1-1811 hinterlegte Stamm ist.

16. Verfahren nach einem der Ansprüche 14 und 15, **dadurch gekennzeichnet, dass** es wenigstens einen Schritt einer Kreuzung umfasst, in welchem der *Agaricus bisporus* der Varietät *burnettii* der bei der CNCM unter der Nr. 1-1812 hinterlegte Stamm ist.

17. Verfahren nach einem der Ansprüche 14 bis 16, **dadurch gekennzeichnet, dass** es wenigstens einen Schritt einer Kreuzung umfasst, in welchem der erste *Agaricus bisporus* unter einem *Agaricus bisporus* der Varietät *burnettii*, einem hybriden Abkömmling dieser Varietät und einem kommerziellen Stamm von *Agaricus bisporus* ausgewählt wird.

18. Verfahren nach Anspruch 17, bei welchem der kommerzielle Stamm die kommerzielle Varietät U1 ist.
